(19) Europäisches Patentamt
European Patent Office
Office européen des brevets

(11) **EP 1 776 971 A1**

(12) # EUROPEAN PATENT APPLICATION

(43) Date of publication:
**25.04.2007 Bulletin 2007/17**

(51) Int Cl.:
*A61M 1/16* (2006.01)   *A61M 1/34* (2006.01)
*A61M 1/36* (2006.01)

(21) Application number: **05425740.7**

(22) Date of filing: **21.10.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **BELLCO S.p.A.**
**20121 Milano (IT)**

(72) Inventors:
• **Albertini, Milco**
**41037 Mirandola (IT)**
• **Baroni, Paolo**
**46035 Ostiglia (IT)**
• **Cavani, Silvia**
**41100 Modena (IT)**
• **Cianciavicchia, Domenico**
**64040 Cavuccio (IT)**
• **Facchini, Mauro**
**41037 Mirandola (IT)**
• **Fiorenzi, Andrea**
**41013 Caselfranco Emilia (IT)**
• **Pradelli, Alessandro**
**41034 Finale Emilia (IT)**

(74) Representative: **Jorio, Paolo et al**
**STUDIO TORTA S.r.l.**
**Via Viotti, 9**
**10121 Torino (IT)**

(54) **Machine for dialysis with improved control of the state of advance of the haemodialysis treatment**

(57) A machine (1; 15; 18; 24) for dialysis comprising: a haemodialysis filter (2); an inlet branch (5) for inlet of a dialysing solution into said filter (2); an outlet branch (6) for outlet of the dialysing solution from said filter (2); a first conductivity meter (7) and a second conductivity meter (8) applied, respectively, on the inlet branch (5) and on the outlet branch (6) for the dialysing solution; an arterial line (3), which is responsible for transport of the blood from the patient to the filter (2); a venous line (4), which is responsible for transport of the blood from the filter (2) to the patient; a plurality of pumps (9, 10) for the circulation both of the blood and of the dialysing solution; a third conductivity meter (8; 14) applied on a ultrafiltered-plasma line (6; 13); and a control unit (11) for data management connected to the conductivity meters (7, 8, 14).

Fig.1

EP 1 776 971 A1

**Description**

The present invention relates to a machine for dialysis with improved control of the state of advance of the haemodialysis treatment.

The dialysis is a method of purification of the blood capable of restoring the hydrosaline balance and of eliminating the water in excess and the toxic substances that accumulate in the organism as a result of renal insufficiency, transferring them to a liquid with electrolytic content similar to that of normal plasma, which does not contain them; here and in what follows said liquid will be designated by the term «dialysing solution». The application of said method envisages that the blood, once drawn from the arm of the patient, traverses the so-called arterial line, and is introduced into the dialyser, at the outlet of which it traverses what is called the venous line and returns purified to the patient.

During haemodialysis there is the need to know significant parameters to enable evaluation of the state of advance of the treatment and, if need be, modification of the conditions of the treatment itself for the purpose of achieving the pre-set therapeutic goal. A parameter that enables appreciation of the advance and, hence, the adequacy of the treatment is the reduction of urea. According to the Gotch's single-compartment model the variation in concentration of the urea in the blood is linked to the dimensionless ratio Kt/V, where K is the clearance of the urea, t is the time of haemodialysis treatment, and V is the volume of distribution of the body water. From various publications it emerges that the clearance of urea K is equivalent to ion dialysance D, defined as the equivalent flow of the ions exchanged by diffusion with the blood of the patient in the dialyser during the process of dialysis. The dialysance is described by the following equation:

$$K \;=\; D \;=\; (Q_d \;+\; Q_{uf}) \;\times\; (C_{do} \;-\; C_{di}) \,/\, (C_{bi} \;-\; C_{di})$$

where Qd and $Q_{uf}$ designate, respectively, the flow of dialysing solution entering the dialyser and the flow of plasmatic water subtracted (ultrafiltered) from the blood, $C_{bi}$ designates the concentration of the solute in the blood entering the dialyser, and $C_{di}$ and $C_{do}$ designate the concentrations of the ions in the dialysing solution entering and leaving the dialyser.

Considering the by now known equivalence between concentrations and conductivity, and given the characteristics of the equipment for dialysis, the measurement of dialysance is generally implemented by means of measurements of conductivity.

Up to the present day, the systems of measurement of dialysance are based on the Polashegg method, where determination of the two unknown quantities D and $C_{bi}$ is made by measuring the conductivity of the dialysing solution at input and at output in two successive steps, characterized by different values of conductivity of the solution entering the dialyser.

Such a methodology suffers from the drawback both regarding the impossibility of making a continuous measurement and regarding the invasive effect due to the modification of the dialysing solution. Finally, the methodology referred to above imposes a certain complexity of the instrumentation, which must be able to make a periodic modification of the dialysing solution.

The aim of the present invention is to obtain a machine for dialysis, the technical characteristics of which will be such as to solve the problems of the known art.

The subject of the present invention is a machine for dialysis comprising: a haemodialysis filter; an inlet branch for inlet of a dialysing solution into said filter; an outlet branch for outlet of the dialysing solution from said filter; a first conductivity meter and a second conductivity meter applied, respectively, on said inlet branch and on said outlet branch for the dialysing solution; an arterial line, which is responsible for transport of the blood from the patient to the filter; a venous line, which is responsible for transport of the blood from the filter to the patient; and a plurality of pumps designed for circulation both of the blood and of the dialysing solution; said machine being characterized in that it comprises a third conductivity meter applied on an ultrafiltered-plasma line and a control unit for data management connected to said conductivity meters.

According to a preferred embodiment, the machine for dialysis forming the subject of the present invention comprises an isolated-ultrafiltration device separated from the haemodialysis filter and set along the arterial line of the blood of the patient; the ultrafilter device is designed to produce the ultrafiltered plasma, on which said third conductivity meter is applied.

According to a further preferred embodiment, the machine for dialysis forming the subject of the present invention comprises means for deviation of flow of the dialysing solution, designed to connect the inlet branch to the outlet branch of the dialysing solution, bypassing the haemodialysis filter, and at the same time said second conductivity meter coincides with said third conductivity meter.

A further subject of the present invention is a machine for dialysis comprising: a haemodialysis filter; an inlet branch for inlet of a dialysing solution into said filter; an outlet branch for outlet of the dialysing solution from said filter; a first conductivity meter and a second conductivity meter applied, respectively, on said inlet branch and on said outlet branch for the dialysing solution; an arterial line, which is responsible for transport of the blood from the patient to the filter; a venous line, which is responsible for transport of the blood from the filter to the patient; and a plurality of pumps designed for circulation both of the blood and of the dialysing solution; said machine being characterized in that it comprises a third conductivity meter applied on said arterial line and/or on said venous line, a haematocritometer applied on said arterial line, and a control unit for data management connected both to said haematocritometer and to said conductivity meters.

The ensuing description is provided by way of illustrative and non-limiting examples in order to facilitate understanding of the invention with the aid of the figures of the annexed drawings, wherein:

- Figure 1 is a schematic view of part of a first preferred embodiment of the machine for dialysis according to the present invention;
- Figure 2 is a schematic view of part of a second embodiment of the machine for dialysis according to the present invention;
- Figure 3 is a schematic view of part of a third embodiment of the machine for dialysis according to the present invention;
- Figure 4 is a schematic view of part of a fourth embodiment of the machine for dialysis according to the present invention;
- Figure 5 is a schematic view of part of a fifth embodiment of the machine for dialysis according to the present invention; and
- Figure 6 is a schematic view of part of a sixth embodiment of the machine for dialysis according to the present invention.

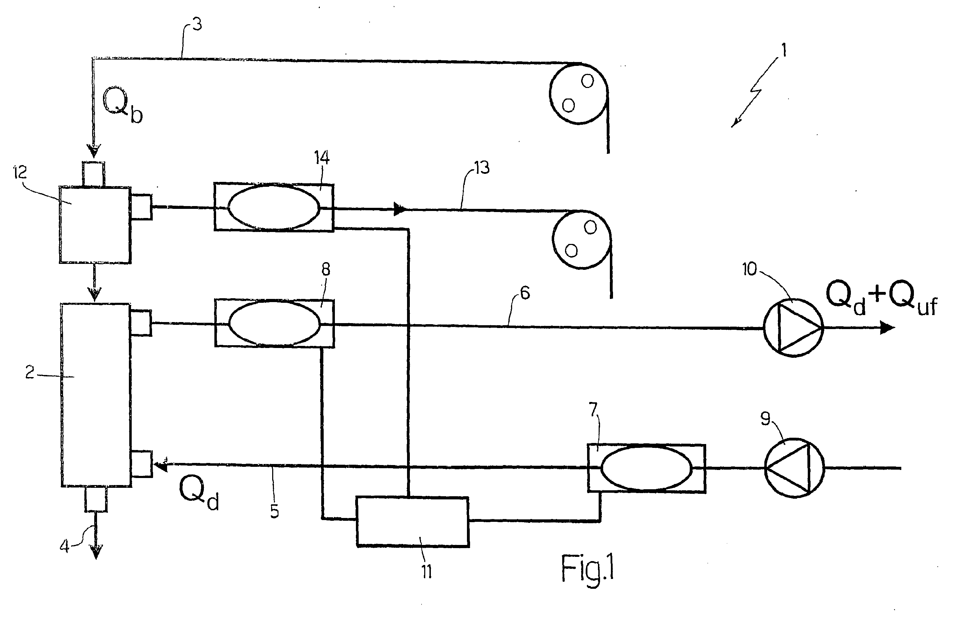

Designated as a whole by 1 in Figure 1 is a machine for dialysis (illustrated only partially), of which there are illustrated schematically: a haemodialysis filter 2 (known and not described in detail); an arterial line 3, which is responsible for transport of the blood from the patient to the filter 2; a venous line 4, which is responsible for transport of the blood from the filter 2 to the patient; an inlet branch 5 and an outlet branch 6 for the dialysing solution, respectively, entering and leaving the filter 2; a pair of conductivity meters 7 and 8, applied, respectively, to the inlet branch 5 and to the outlet branch 6 for the dialysing solution; a first pump 9 and a second pump 10 applied, respectively, to the inlet branch 5 and to the outlet branch 6 for the dialysing solution; and a control unit for reading and processing 11, designed to receive information from the conductivity meters 7 and 8 and processing their data.

The machine for dialysis 1 comprises an isolated-ultrafiltration device 12 set along the arterial line 3 in such a way as to produce a quantity of ultrafiltered plasma not yet subjected to haemodialysis. The ultrafiltered plasma is introduced along a arterial branching line 13, which generally comprises a device for purification by adsorption and reconnects to the arterial line 3 in the stretch comprised between the isolated-ultrafiltration device 12 itself and the haemodialysis filter 2. The machine 1 moreover comprises a third conductivity meter 14, applied to the arterial branching line 13 and connected to the control unit for reading and processing 11. In this way, in addition to enabling continuous reading of the conductivity of the dialysing solution entering and leaving the haemodialysis filter 2 by means of the conductivity meters 7 and 8, it also enables continuous reading of the conductivity of the ultrafiltered plasma by means of the conductivity meter 14.

In this way, it is no longer necessary to use two different dialysing solutions with all the problems that this entails, and it is easy to derive the value of D and, hence, of the dimensionless ratio Kt/V, by introducing the following equation into the control unit for reading and processing 11:

$$K = D = (Q_d + Q_{uf}) \times (C_{domis} - C_{dimis}) / (C_{plasmin} - C_{dimis})$$

where $C_{dimis}$ is the conductivity for the dialysing solution entering the filter 2 and measured with the conductivity meter 7, $C_{domis}$ is the conductivity of the dialysing solution leaving the filter 2 and measured with the conductivity meter 8, $C_{plasmin}$ is the conductivity of the plasma entering the filter 2 and measured with the conductivity meter 14, whilst $Q_d$ and $Q_{uf}$, as given above, designate, respectively, the flow of dialysing solution entering the dialyser and the flow of water of the plasma (ultrafiltered) subtracted from the blood.

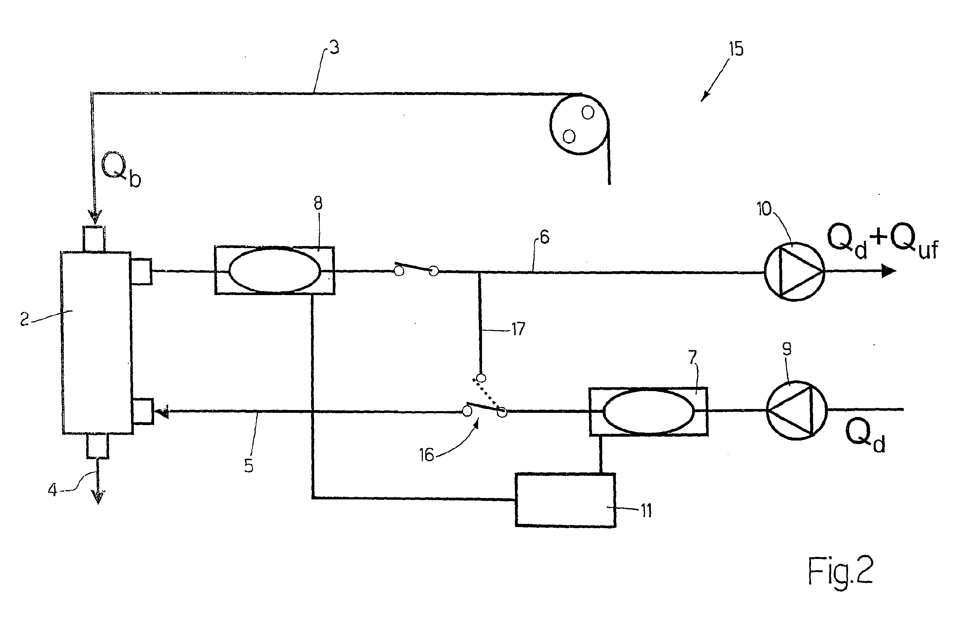

Designated as a whole by 15 in Figure 2 is a second preferred embodiment of a machine for dialysis (illustrated only partially), the parts of which that are identical to the ones of the machine 1 will be designated by the same reference numbers and will not be described again herein.

The machine 15, as compared to the machine 1, is without the isolated-ultrafiltration device 12 of the branching line 13 and of the conductivity meter 14. The machine 15 comprises a flow deviator 16 set on the inlet branch 5 downstream of the conductivity meter 7 with respect to the direction of flow for the dialysing solution, and a connection branch 17, designed to connect the inlet branch 5 with the outlet branch 6. In particular, the connection branch 17 has a first end connected to the flow deviator 16 and a second end coupled to the outlet branch 6 in a coupling point set downstream of the conductivity meter 8. In this way, it is possible to deviate the flow of the dialysing solution, causing it to pass from the inlet branch 5 directly to the outlet branch 6, bypassing the filter 2. When the dialysing solution is deviated as described above, the filter 2 executes only an isolated ultrafiltration in such a way that the conductivity meter 8 is able to measure the conductivity of the ultrafiltered plasma alone.

In other words, in the machine for dialysis 15 the conductivity meter 8 measures the $C_{plasmin}$ or the $C_{domis}$ according to whether the flow deviator 16 is activated or not. In this way, using the conductivity meters 7 and 8 it is possible to derive the dimensionless ratio Kt/V, as described for the machine 1.

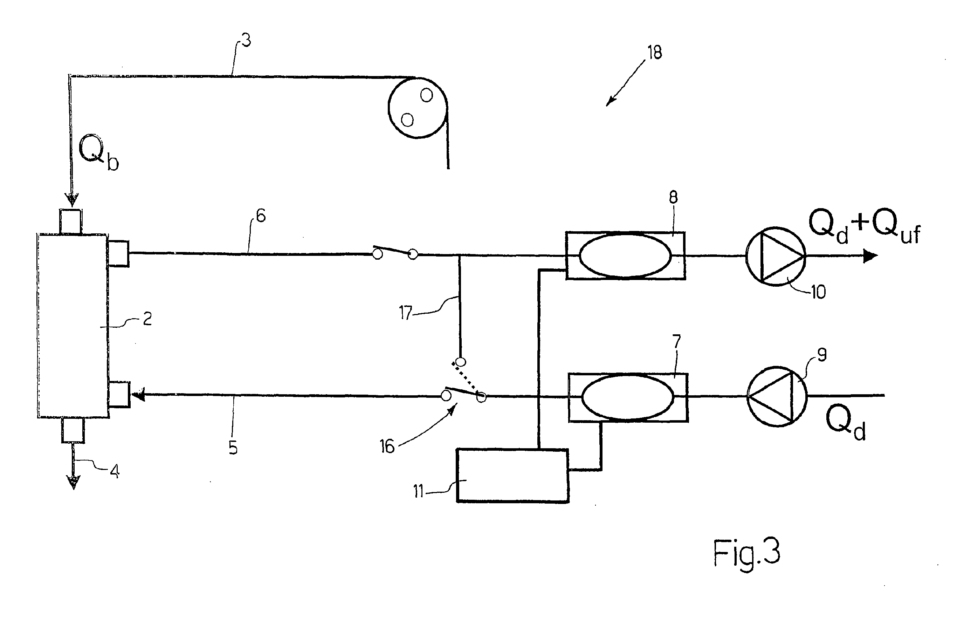

In Figure 3, designated as a whole by 18 is a third preferred embodiment of a machine for dialysis (illustrated only partially), which differs from the machine 15 exclusively in that the conductivity meter 8 is set downstream instead of upstream of the point where the connection branch 17 is coupled to the outlet branch 6.

In this case, as may readily be appreciated, the conductivity of the blood entering the filter is not measured directly, but is obtained from the following equation:

$$C_{plasmin} = (Q_{uf}/Q_d + 1) \ x \ C_{dois} - Q_{uf}/Q_d \ x \ C_{dimis}$$

where $C_{dois}$ is the measurement of conductivity measured by the conductivity meter 8 when the flow deviator 16 is activated, and hence the filter performs only an isolated ultrafiltration.

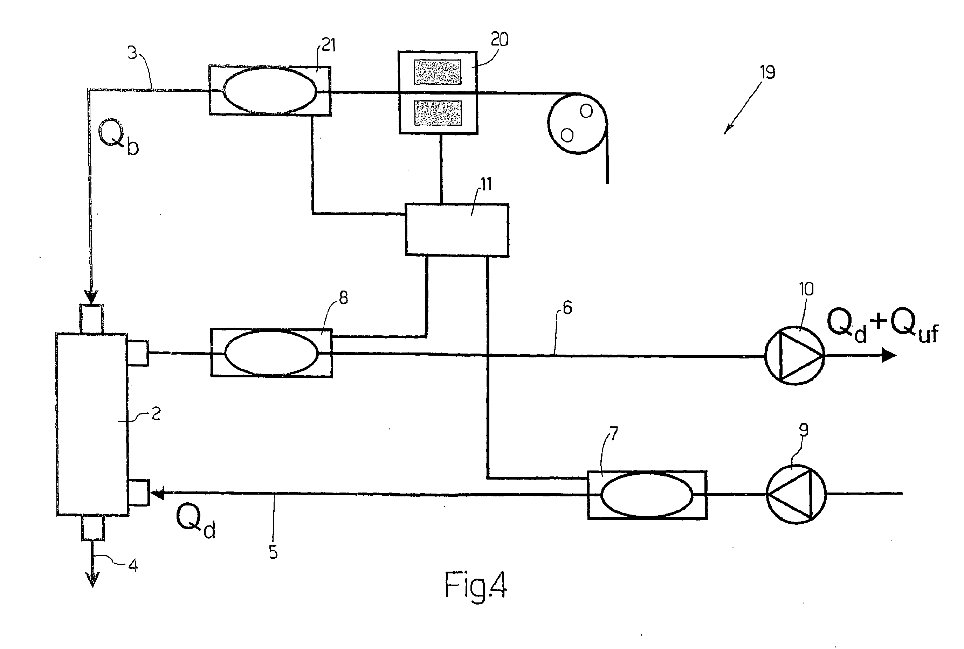

Designated as a whole by 19 in Figure 4 is a fourth preferred embodiment of a machine for dialysis (illustrated only partially), the parts of which that are identical to the ones of the machine 1 will be designated by the same reference numbers and will not be described again herein.

The machine 19 comprises, along the arterial line 3, a haematocritometer 20 and a conductivity meter 21 for measuring the conductivity of blood. In the machine 19, the conductivity of the plasma is obtained from the conductivity of the blood entering the filter 2, from the haematocrit (Hct), and from the characteristics of the patient according to a particular function that will be entered into the control unit 11: $C_{plasmin} = f(C_{bin}, Hct_{in}, \text{patient})$, where $C_{bin}$ is the conductivity of the blood entering the filter 2.

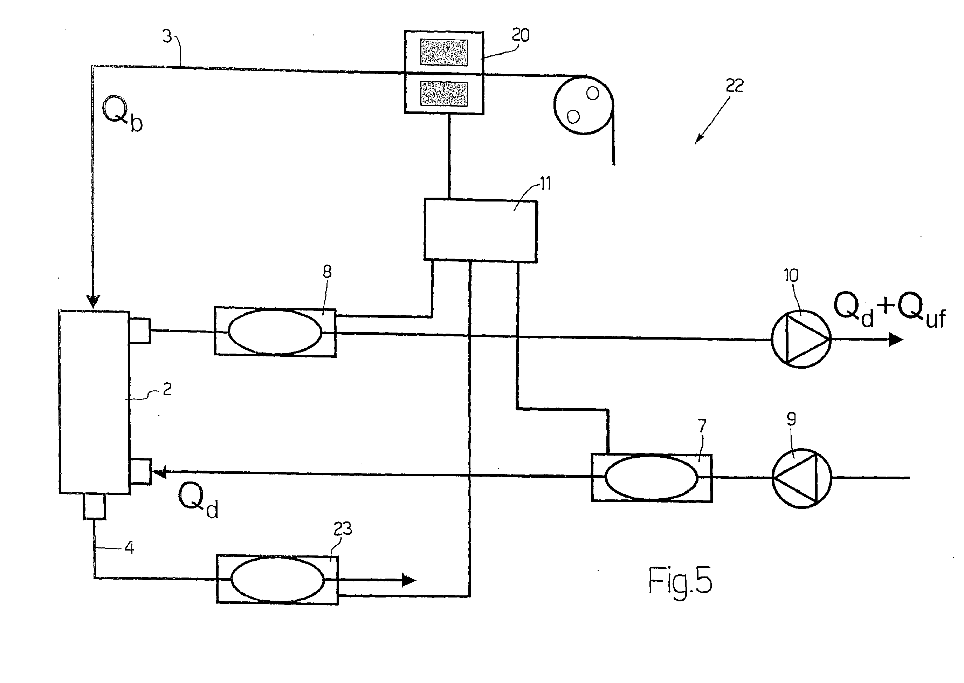

Designated as a whole by 22 in Figure 5 is a fifth preferred embodiment of a machine for dialysis (illustrated only partially), the parts of which that are identical to the ones of the machine described previously will be designated by the same reference numbers and will not be described again herein.

The machine 22 differs from the machine 19 in that it comprises a conductivity meter 23 for measuring the conductivity of the blood set along the venous line 4 instead of along the arterial line 3.

In this case, the dialysance, and hence the clearance is calculated by entering the following equation into the control unit 11:

$$K = D = (Q_b - Q_{uf}) \ x \ (C_{plasmin} - C_{plasmout})/(C_{plasmin} - C_{dimis}) + Q_{uf}$$

where:

$Q_b$ is the blood flow entering the filter 2;

$C_{plasmout}$ is the conductivity of the plasma leaving the filter 2 and is calculated from the conductivity of the blood leaving the filter 2, from the haematocrit $Hct_{out}$ and from the characteristics of the patient according to the particular function that will be entered into the control unit 11: $C_{plasmout} = f(C_{bo}, Hct_{out}, \text{patient})$, where $C_{bo}$ is the conductivity of the blood leaving the filter 2 and measured by the conductivity meter 23 of the blood, whilst $Hct_{out}$ is the value of the haematocrit of the blood leaving the filter 2 and is calculated in turn using the equation

$$Hct_{out} = H_{ctin} \ x \ Q_b/(Q_b - Q_{uf})$$

entered into the control unit 11 and where Hct$_{in}$ is measured by the haematocritometer 20; and C$_{plasmin}$ is calculated applying the equation

$$C_{plasmin} = ((Q_b - Q_{uf}) \times C_{plasmout} + (Q_d + Q_{uf}) \times C_{domis} - Q_d \times C_{dimis})/Q_b.$$

entered into the control unit 11.

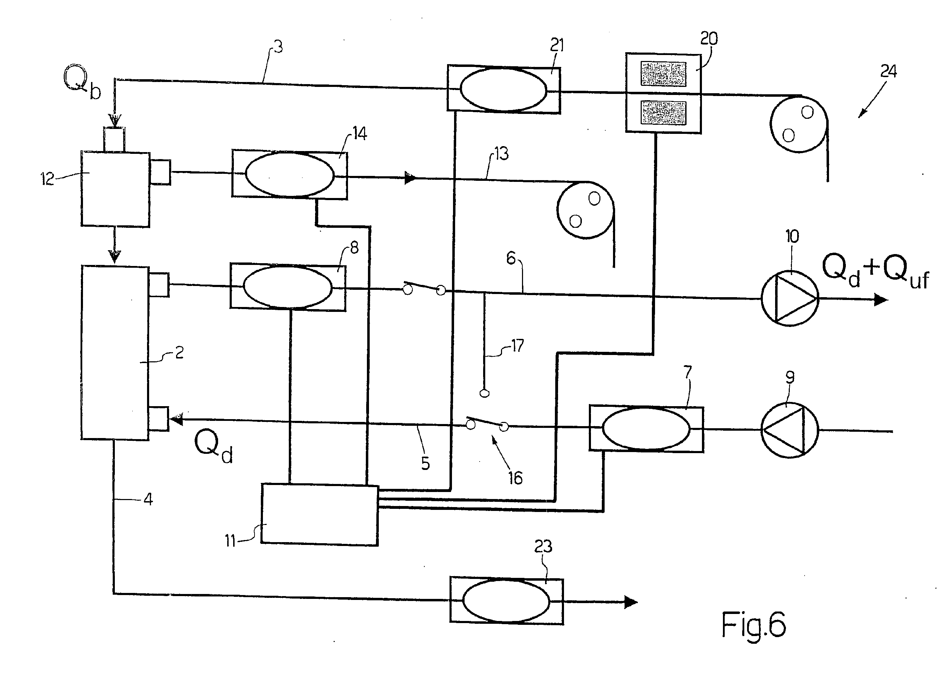

Designated as a whole by 24 in Figure 6 is a sixth preferred embodiment of a machine for dialysis (illustrated only partially). The machine 24 comprises all the devices belonging to the machine of the present invention described previously. In other words, the machine 24 enables calculation of the dimensionless ratio Kt/V by measuring the conductivity of the plasma either directly, using the conductivity meter 14 or the conductivity meter 8, or indirectly, using the conductivity meter 8 set downstream instead of upstream of the point where the connection branch 17 is coupled to the outlet branch 6, or else the blood-conductivity meters 21 or 23 and the haematocritometer 20.

In this way, it will be possible to compare the different methods and hence carry out checks and verifications in order to gain an increasingly better control of the state of advance of the haemodialysis treatment.

The machine according to the present invention enables measurement in a continuous, or practically continuous, way of the conductivity of the plasma, and hence of the dimensionless parameter Kt/V, enabling an evaluation as faithful as possible of the haemodialysis treatment, with the consequence of enabling intervention in a more efficient way.

Furthermore, it is envisaged that the machine of the present invention will have entered into the control unit 11 a system of intervention on the dialysing solution as a function of the values of the dimensionless parameter Kt/V derived. For said purpose, the control unit 11, in addition to being connected to the conductivity meters, will be connected to the pumps and to a device for preparation of the dialysing solution.

In this way, the machine according to the present invention affords a further advantage consisting in the automatic adjustment of the dialysing solution.

Finally, as will be obvious to a person skilled in the branch, the machine according to the present invention can be adapted to any technique for dialysis such as, for example, that of haemodiafiltration.

## Claims

1. A machine (1; 15; 18; 24) for dialysis comprising: a haemodialysis filter (2); an inlet branch (5) for inlet of a dialysing solution into said filter (2); an outlet branch (6) for outlet of the dialysing solution from said filter (2); a first conductivity meter (7) and a second conductivity meter (8), applied, respectively, on said inlet branch (5) and on said outlet branch (6) for the dialysing solution; an arterial line (3), which is responsible for transport of the blood from the patient to the filter (2); a venous line (4), which is responsible for transport of the blood from the filter (2) to the patient; and a plurality of pumps (9, 10) designed for the circulation both of the blood and of the dialysing solution; said machine being **characterized in that** it comprises a third conductivity meter (8; 14) applied on an ultrafiltered-plasma line (6; 13) and a control unit (11) for data management connected to said conductivity meters (7, 8, 14).

2. The machine for dialysis according to Claim 1,
   **characterized in that** it comprises a device (12) for isolated ultrafiltration set along the arterial line (3) of the blood of the patient, said third conductivity meter (14) being applied to the ultrafiltered plasma produced by said ultrafilter device (12).

3. The machine for dialysis according to Claim 1,
   **characterized in that** it comprises means of deviation of the flow (16, 17) of the dialysing solution, designed, if activated, to connect the inlet branch (5) to the outlet branch (6) for the dialysing solution, bypassing the haemodialysis filter; said second conductivity meter (8) coinciding with said third conductivity meter (8).

4. The machine for dialysis according to Claim 3,
   **characterized in that** said means of deviation of flow comprise a connection branch (17), one end of which is coupled to the outlet branch (6) of the dialysing solution in a position downstream of said second conductivity meter (8) in relation to the flow of the dialysing solution.

5. The machine for dialysis according to Claim 3,
   **characterized in that** said means of deviation of flow comprise a connection branch (17), one end of which is coupled to the outlet branch (6) of the dialysing solution in a position upstream of said second conductivity meter (8) in relation to the flow of the dialysing solution.

6. The machine for dialysis according to any one of the preceding claims, **characterized in that** it comprises a fourth conductivity meter (21; 23) applied on said arterial line (3) and/or on said venous line (4) and a haematocritometer (20) applied on said arterial line (3); said fourth conductivity meter (21; 23) and said haematocritometer (20) being connected to said control unit (11) for data management.

7. A machine (19, 22) for dialysis comprising: a haemodialysis filter (2); an inlet branch (5) for inlet of a dialysing solution into said filter (2); an outlet branch (6) for outlet of the dialysing solution from said filter (2); a first conductivity meter (7) and a second conductivity meter (8) applied, respectively, on said inlet branch (5) and on said outlet branch (6) for the dialysing solution; an arterial line (3), which is responsible for transport of the blood from the patient to the filter (2); a venous line (4), which is responsible for transport of the blood from the filter (2) to the patient; and a plurality of pumps (9, 10) designed for circulation both of the blood and of the dialysing solution; said machine being **characterized in that** it comprises a third conductivity meter (21; 23) applied on said arterial line (3) and/or on said venous line (4), a haematocritometer (20) applied on said arterial line (3), and a control unit (11) for data management connected both to said haematocritometer (20) and to said concentration meters (7, 8, 21; 23).

Fig.1

Fig.2

$Q_b$

$Q_d+Q_{uf}$

$Q_d$

2

3

4

5

6

7

8

9

10

11

15

16

17

EP 1 776 971 A1

Fig.3

Fig.4

EP 1 776 971 A1

Fig.5

Fig.6

**European Patent Office**

## EUROPEAN SEARCH REPORT

Application Number

EP 05 42 5740

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 34 39 661 A1 (FRESENIUS AG; FRESENIUS AG, 6380 BAD HOMBURG, DE) 7 May 1986 (1986-05-07) | 1,3,4 | A61M1/16 A61M1/34 A61M1/36 |
| Y | * page 19, line 21 - line 24 * * page 20, line 34 - page 21, line 1 * * page 26, line 21 - line 24; figures 2,3 * | 2,5,6 | |
| Y | EP 0 693 297 A (BELLCO S.P.A) 24 January 1996 (1996-01-24) * column 2, line 25 - line 40 * * column 3, line 11 - line 16; figure 1 * | 2 | |
| Y | US 5 342 527 A (CHEVALLET ET AL) 30 August 1994 (1994-08-30) * column 4, line 29 - line 45; figure 2 * | 5 | |
| Y | EP 0 272 414 A (FRESENIUS AG) 29 June 1988 (1988-06-29) | 6 | |
| X | * page 2, line 55 - page 3, line 12 * * page 5, line 20 - line 23; figure 1 * | 7 | |

TECHNICAL FIELDS SEARCHED (IPC)

A61M

The present search report has been drawn up for all claims

| Place of search | Date of completion of the search | Examiner |
|---|---|---|
| Munich | 14 March 2006 | Böttcher, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

EP 1 776 971 A1

## ANNEX TO THE EUROPEAN SEARCH REPORT
## ON EUROPEAN PATENT APPLICATION NO.

EP 05 42 5740

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2006

| Patent document cited in search report | | Publication date | Patent family member(s) | | Publication date |
|---|---|---|---|---|---|
| DE 3439661 | A1 | 07-05-1986 | NONE | | |
| EP 0693297 | A | 24-01-1996 | IT | B0940336 A1 | 18-01-1996 |
| US 5342527 | A | 30-08-1994 | CA | 2099056 A1 | 31-12-1993 |
| | | | DE | 69300078 D1 | 13-04-1995 |
| | | | DE | 69300078 T2 | 31-08-1995 |
| | | | EP | 0579559 A1 | 19-01-1994 |
| | | | ES | 2071534 T3 | 16-06-1995 |
| | | | FR | 2692983 A1 | 31-12-1993 |
| | | | JP | 3274742 B2 | 15-04-2002 |
| | | | JP | 6254156 A | 13-09-1994 |
| EP 0272414 | A | 29-06-1988 | DE | 3640089 A1 | 01-06-1988 |
| | | | ES | 2026508 T3 | 01-05-1992 |
| | | | JP | 2532261 B2 | 11-09-1996 |
| | | | JP | 63143077 A | 15-06-1988 |

EPO FORM P0459

For more details about this annex : see Official Journal of the European Patent Office, No. 12/82